# FASCICULE DE BREVET EUROPEEN

(11) **EP 0 562 659 B1**
(45) Date de publication et mention de la délivrance du brevet: **17.12.1997**
(21) Numéro de dépôt: 93200690.1
(22) Date de dépôt: 10.03.1993
(51) Int. Cl.: C07K 1/00, C07K 1/06, C07K 5/08, C07C 271/22, C07K 1/107

(54) **Procédé de synthèse peptidique et nouveaux intermédiaires de synthèse**
Verfahren zur Herstellung von Peptiden und neue Zwischenverbindungen dazu
Process to synthetise peptides and new intermediates thereof

(30) Priorité: 20.03.1992 BE 9200273
(43) Date de publication de la demande: 29.09.1993
(73) Titulaire: SOLVAY (Société Anonyme), B-1050 Bruxelles (BE)
(72) Inventeur: Anteunis, Marc, B-9030 Mariakerke (BE); Becu, Frank, B-8490 Jabbeke (BE); Callens, Roland, B-9031 Drongen (BE); Blondeel, Georges, B-9300 Aalst (BE)
(74) Mandataire: Jacques, Philippe

(56) Documents cités:
- BE-A- 693 604
- HELVETICA CHIMICA ACTA vol. 36, no. 4, 15 Juin 1953, BASEL CH pages 875 - 886 R A BOISSONNAS ET AL. 'Etude comparative de la scission de divers groupes de blocage de la fonction alpha-amino des acides aminés'

## Description

La présente invention se rapporte à un nouveau procédé de synthèse de peptides contenant un ou plusieurs restes d'aminoacides comportant une fonction N-carbamoyle. L'invention concerne aussi de nouveaux intermédiaires de synthèse mis en oeuvre dans ce procédé et de nouveaux fragments peptidiques.

Divers peptides synthétiques, utilisés notamment comme réactifs pour des tests analytiques, comme agents édulcorants, code hormones synthétiques ou code médicaments, renferment des restes d'aminoacides comportant une fonction N-carbamoyle, soit sur la fonction α-amino du reste N-terminal, soit sur la fonction ω-amino d'un reste de diaminoacide, c'est-à-dire en bout de chaîne latérale. De tels diaminoacides sont notamment la norcitrulline (Nci), la citrulline (Cit) ou l'homocitrulline (Hci).

L'introduction d'un reste d'aminoacide comportant une fonction N-carbamoyle dans une chaîne peptidique, directement au départ de l'acide aminé porteur de cette fonction carbamoyle ne va pas sans inconvénient. En effet, la présence d'une fonction uréido ou uréino perturbe gravement les techniques connues de synthèse peptidique, tant en phase "solide" selon la technique dite de Merrifield, qu'en phase liquide. Les rendements chimiques sont médiocres et une pureté chirale convenable n'est obtenue qu'au prix de purifications laborieuses et onéreuses. De plus, dans le cas des diaminoacides, la synthèse d'acides aminés comportant une fonction uréido ou uréino en bout de chaîne latérale sous une forme énantiomorphe pure est difficile et donc coûteuse.

La synthèse d'un peptide renfermant un reste d'aminoacide comportant une fonction N-carbamoyle est dès lors généralement effectuée en deux stades. Dans une première étape est synthétisé un peptide précurseur comprenant le reste d'aminoacide comportant la fonction amino à carbamoyler. Dans un second temps, la fonction amino à carbamoyler est transformée, au sein de la chaîne peptidique, en fonction uréino.

Il est par exemple bien connu de transformer en fonction uréido, la fonction α-amino du reste valine N-terminal de la chaîne β de l'hémoglobine dépranocytaire, par carbamoylation au moyen de l'isocyanate de sodium (Lehninger A.L. ; Biochemistry ; (1975) second Edition, p. 149).

Ce procédé connu n'apparaît cependant pas satisfaisant en synthèse peptidique, principalement en raison d'une spécificité insuffisante des cyanates ou isocyanates pour la ou les fonctions amino à carbamoyler et en raison de la racémisation qu'un tel traitement peut induire.

D'autre part, R.A. Boissonnas et G. Preitner (Helvetica Chimica Acta, (1953), Vol.36 (4), p.875-886) ont étudié l'élimination des groupements phényloxycarbonyle (PhOC) et p-tolyloxycarbonyle (TOC), utilisés comme groupement de blocage de la fonction α-amino de certains α-aminoacides, au moyen de différents agents de scission. Tous les agents de scission mis en oeuvre ont conduit à l'élimination complète de ces groupements et à la libération de la fonction α-amino.

L'invention remédie aux inconvénients des procédés de préparation de N-carbamoyl-peptides connus en fournissant un procédé nouveau pour préparer des N-carbamoyl-peptides avec un rendement chimique amélioré, permettant de conserver remarquablement la pureté chirale des structures mises en oeuvre.

En conséquence, l'invention concerne un procédé d'obtention d'un peptide comportant une ou plusieurs fonctions N-carbamoyle, selon lequel on fait réagir un peptide intermédiaire comportant un ou plusieurs groupements aryloxycarbonyle constitués par un groupement de formule générale 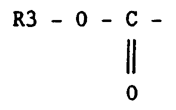 fixé sur l'atome d'azote d'une fonction amino et dans laquelle R3 représente un groupement aryle, substitué ou non par un ou plusieurs groupements alkyles comportant de 1 à 4 atomes de carbone, avec un composé de formule générale R1R2NH dans laquelle R1 et R2 représentent, indépendamment l'un de l'autre, des atomes d'hydrogène, des radicaux alkyles, cycloalkyles ou aralkyles, contenant au maximum 12 atomes de carbone, ou dans laquelle R1 et R2 forment ensemble un radical alicyclique contenant de 3 à 6 atomes de carbone.

Par peptide, on entend désigner, aux fins de la présente invention, tout composé naturel ou synthétique, constitué par l'association d'au moins deux acides aminés naturels ou synthétiques et plus particulièrement associés par une liaison amide. Par extension, on entend aussi englober ci-après sous le vocable "peptide", toute structure peptidique dont certaines fonctions sont éventuellement substituées par des groupements protecteurs ou des groupements d'activation.

Par aminoacide, on entend désigner ci-après tout acide organique possédant au moins une fonction carboxyle et au moins une fonction amino primaire ou secondaire, tel que les acides aminés naturels ou les acides aminés synthétiques. Par extension, on entend aussi englober ci-après sous le vocable "aminoacide", tout aminoacide dont certaines fonctions sont éventuellement substituées par des groupements protecteurs ou des groupements d'activation. Les aminoacides sont de préférence ceux comportant au moins un atome de carbone porteur à la fois d'une fonction carboxyle et d'une fonction amino.

Par fonction N-carbamoyle, on entend désigner ci-après toute fonction carbamoyle -CO-NH₂ fixée sur l'atome d'azote d'une fonction amino d'un aminoacide ou d'un peptide. Par extension, on entend aussi englober ci-après sous le vocable "carbamoyle", toute fonction carbamoyle dont au moins un des deux atomes d'hydrogène est substitué par un radical alkyle, cycloalkyle ou aralkyle contenant au maximum 12 atomes de carbone ou dont les deux atomes d'hydrogène sont substitués par un radical bivalent alicyclique contenant de 3 à 6 atomes de carbone. La fonction carbamoyle forme, avec l'atome d'azote auquel elle est attachée, une fonction uréino ou uréido, selon que la fonction carbamoyle est substituée ou non.

Dans le composé de formule générale R1R2NH, par radical alkyle, cycloalkyle, aralkyle ou alicyclique, on entend désigner, aux fins de la présente invention, aussi bien des radicaux exclusivement hydrocarbonés, que des radicaux hydrocarbonés substitués par des groupements fonctionnels comprenant au moins un atome d'oxygène, de soufre ou d'azote, tels que des groupements carboxyles, hydroxyles, sulfhydryles, indolyles et imidazolyles.

Dans la suite de la description, de même qu'on désigne par fonction N-carbamoyle toute fonction carbamoyle -CO-NH₂ fixée sur l'atome d'azote d'une fonction amino d'un aminoacide ou d'un peptide, on désignera par N-aryloxycarbonyle tout groupement aryloxycarbonyle R3-O-CO- fixé sur l'atome d'azote d'une fonction amino d'un aminoacide ou d'un peptide.

Dans le procédé selon l'invention, la nature de R3 est critique. En effet, il est apparu, de manière surprenante, que contrairement aux groupements protecteurs classiques de la fonction amino, tels que par exemple, le groupement N-benzyloxycarbonyle ou le groupement N-phtaloyle, lesquels, en présence d'ammoniac ou d'une amine, libèrent la fonction amino inaltérée, les groupements N-aryloxycarbonyle conduisent, en présence de tels composés, à la formation d'une fonction N-carbamoyle. Généralement, R3 est un groupement contenant au maximum 12 atomes de carbone. Le plus souvent R3 est un groupement phényle, naphtyle, tolyle, xylyle, mésitylyle, éthylphényle, diéthylphényle, propylphényle ou isopropylphényle. De préférence, R3 est un groupement phényle ou tolyle. De manière particulièrement préférée, R3 est un groupement phényle ou p-tolyle. Dans ce dernier cas, le peptide intermédiaire mis en oeuvre dans le procédé selon l'invention est respectivement le dérivé N-phényloxycarbonyle (PhOC) ou le dérivé N-tolyloxycarbonyle (TOC). Par analogie avec les dénominations PhOC et TOC, les différents groupements aryloxycarbonyle utilisables dans le procédé selon l'invention seront désignés ci-après par l'abréviation générique ArOC.

D'excellents résultats ont été obtenus dans le procédé selon l'invention avec des composés R1R2NH dont les radicaux R1 et R2 sont des atomes d'hydrogène ou des radicaux exclusivement hydrocarbonés. De tels composés sont, par exemple, l'ammoniac ou les amines primaires ou secondaires, choisies parmi les alkylamines, les cycloalkylamines, les aralkylamines et les amines hétérocycliques. Ces amines contiennent en général au maximum 12 atomes de carbone. A titre d'exemples non limitatifs, on peut citer, comme alkylamines, la méthylamine, l'éthylamine, la n-propylamine, l'isopropylamine, la n-butylamine, la tert-butylamine, la 2-méthylbutylamine, la diméthylamine, la diéthylamine et la N-méthyl-N-éthylamine, comme cycloalkylamines, la cyclopentylamine et la cyclohexylamine, comme aralkylamines, la benzylamine, la N-(phényléthyl)-amine, la N-(phénylpropyl)-amine et la N-(phénylbutyl)-amine, comme amines hétérocycliques, la pyrrolidine et la pipéridine.

A titre d'exemples de composés R1R2NH dont les radicaux R1 et/ou R2 sont des radicaux hydrocarbonés substitués par des groupements fonctionnels comprenant au moins un atome d'oxygène, de soufre ou d'azote, on peut citer notamment les acides aminés, tels que définis plus haut.

Dans le cas où le composé R1R2NH est l'ammoniac, la fonction N-carbamoyle formée par le procédé selon l'invention est non substituée et le peptide obtenu comporte donc une fonction uréido. Dans tous les autres cas (R1 et/ou R2 différent(s) de H), la fonction N-carbamoyle formée par le procédé selon l'invention est substituée par au moins un groupement contenant un ou plusieurs atomes de carbone et le peptide obtenu comporte alors une fonction uréino.

Dans un peptide, 2 types de fonction amino sont susceptibles d'être carbamoylées : d'une part, la fonction α-amino du reste N-terminal et, d'autre part, la fonction ω-amino d'un reste de diaminoacide, quelle que soit la position de ce diaminoacide au sein de la chaîne peptidique.

Le procédé selon l'invention permet de préparer des N^{α}-carbamoyl-peptides et des N^{ω}-carbamoyl-peptides. Par N^{α}-carbamoyl-peptide, on entend désigner tout peptide comportant une fonction carbamoyle, telle que définie plus haut, sur la fonction α-amino du reste N-terminal d'une chaîne peptidique. Par N^{ω}-carbamoyl-peptide, on entend désigner tout peptide comportant une fonction carbamoyle, telle que définie plus haut, sur une ou plusieurs fonctions ω-amino de tout reste de diaminoacide situé en n'importe quelle position de la chaîne peptidique.

Le procédé selon l'invention apparaît particulièrement performant pour l'obtention de N^{ω}-carbamoyl-peptides au départ d'un peptide intermédiaire comportant un groupement aryloxycarbonyle sur une ou plusieurs fonctions ω-amino. De manière préférée, le procédé selon l'invention est mis en oeuvre pour transformer, au sein d'une structure peptidique, un reste lysine, ornithine ou 2,4-diaminobutyrique dont la fonction ω-amino en bout de chaîne latérale est substituée par un groupement ArOC, respectivement en reste homocitrulline, citrulline ou 2-amino-4-uréido-butyrique (norcitrulline), quelle que soit leur configuration (D, L ou DL).

Le peptide intermédiaire comportant un ou plusieurs groupements N-aryloxycarbonyle que l'on met en oeuvre dans le procédé selon l'invention peut, en outre, comporter d'autres fonctions substituées par des groupements protecteurs ou des groupements d'activation.

Par groupement protecteur ou groupement d'activation, on entend tout composé cité à cet effet dans la littérature. A titre illustratif de tels groupements protecteurs ou d'activation pouvant substituer une ou plusieurs fonctions du peptide intermédiaire comportant un ou plusieurs groupements N-aryloxycarbonyle mis en oeuvre dans le procédé selon l'invention, on peut citer :
a) Comme groupements protecteurs de la fonction amino,
   - des groupements de type alkyle ou aralkyle, substitués ou non, comportant des hétéroatomes ou non, tels que les groupements benzyle, diphénylméthyle, di(méthoxyphényl)méthyle et triphénylméthyle (trityle),
   - des groupements de type acyle, substitués ou non, tels que notamment les groupements formyle, acétyle, trifluoro-acétyle, benzoyle et phtaloyle,
   - des groupements de type aralkyloxycarbonyle, substitués ou non, tels que notamment les groupements benzyloxycarbonyle, p-chlorobenzyloxycarbonyle, p-bromobenzyloxycarbonyle, p-nitrobenzyloxycarbonyle, p-méthoxybenzyloxycarbonyle, benzhydryloxycarbonyle, 2-(p-biphénylyl)isopropyloxycarbonyle, 2-(3,5-diméthoxyphényl)isopropyloxycarbonyle, p-phénylazobenzyloxycarbonyle, triphénylphosphonoéthyloxycarbonyle et 9-fluorénylméthyloxycarbonyle,
   - des groupements de type alkyloxycarbonyle, substitués ou non, tels que notamment les groupements tert-butyloxycarbonyle, tert-amyloxycarbonyle, diisopropylméthyloxycarbonyle, isopropyloxycarbonyle, éthyloxycarbonyle, allyloxycarbonyle, 2-méthylsulfonyléthyloxycarbonyle et 2,2,2-trichloroéthyloxycarbonyle,
   - des groupements de type cycloalkyloxycarbonyle tels que notamment les groupements cyclopentyloxycarbonyle, cyclohexyloxycarbonyle, adamantyloxycarbonyle et isobornyloxycarbonyle,
   - des groupements dérivés d'hétéroatomes tels que les groupements benzènesulfonyle, p-toluenesulfonyle (tosyle), mésitylènesulfonyle, méthoxytriméthylphénylsulfonyle, o-nitrophénylsulfényle et triméthylsilyle,
b) Comme groupements protecteurs de la fonction carboxyle, des groupements tels que notamment les groupements méthyle, éthyle, t-butyle, cyclohexyle, benzyle, p-nitrobenzyle, phénacyle, triméthylsilyle, carboxamidométhyle et fluorénylméthyle,
c) Comme groupements protecteurs de la fonction hydroxyle, des groupements tels que notamment les groupements t-butyle, tétrahydropyranyle, benzyle, triméthylsilyle, méthoxyméthyle, trityle et fluorénylméthyle,
d) Comme groupements d'activation, notamment les groupements O-succinimidoyle, O-norbornènedicarboximidoyle, O-benzotriazolyle, O-oxodihydrobenzotriazolyle, O-hydroxypyridyle, pentachlorophényle, trichlorophényle, pentafluorophényle, isobutyloxycarbonyle et triméthylacétyle.

Dans un premier mode d'exécution du procédé d'obtention d'un N-carbamoyl-peptide selon l'invention, le peptide intermédiaire comportant un ou plusieurs groupements N-ArOC est synthétisé au départ du dérivé N-ArOC d'un aminoacide, c'est-à-dire par incorporation au sein de la chaîne peptidique de l'aminoacide dont on souhaite transformer une fonction amino en fonction uréido ou uréino, directement sous la forme de son dérivé N-ArOC. Il est en effet apparu, de manière surprenante, que ces dérivés N-ArOC des acides aminés sont non seulement remarquablement stables, mais de plus, très respectueux de la chiralité des composés, ce qui permet leur mise en oeuvre dans les procédés chimiques classiques de synthèse peptidique, tant en phase solide qu'en phase liquide. L'utilisation, comme réactif de synthèse du peptide intermédiaire, de l'acide aminé N-substitué par le groupement ArOC s'avère particulièrement avantageuse. En effet, le groupement ArOC assurant de manière remarquablement efficace le rôle de groupement protecteur de la fonction amino en question, conjointement à un rôle d'intermédiaire particulièrement spécifique de la fonction uréido ou uréino recherchée tout en respectant la chiralité des composés, ce mode de mise en oeuvre du procédé selon l'invention permet de préparer le N-carbamoyl-peptide souhaité avec un rendement encore jamais atteint.

Le dérivé N-ArOC de l'acide aminé dont on souhaite obtenir in fine le dérivé N-carbamoyle au sein d'un peptide peut être préparé classiquement par une méthode similaire à celles utilisées pour fixer sur une fonction amino un groupement protecteur de type alkyloxycarbonyle ou aralkyloxycarbonyle. Une méthode classique, connue comme la procédure de Schotten-Baumann consiste à faire réagir, en milieu aqueux, la forme sodique de l'acide aminé avec le chloroformiate d'aryle souhaité. Une autre procédure qui a conduit à d'excellents résultats et qui est dès lors préférée, consiste à mettre en oeuvre l'acide aminé sous la forme de son dérivé persilylé. Ce dérivé persilylé peut être obtenu par exemple par un traitement à reflux avec un excès de triméthylcyanosilane, jusqu'à obtention d'une solution homogène. Cette solution est ensuite diluée, par exemple par du chlorure de méthylène, et refroidie à une température inférieure à -10 °C, de préférence inférieure ou égale à -15 °C. La quantité stoechiométrique de chloroformiate d'aryle est alors ajoutée très lentement, puis après quelques minutes de réaction, la solution est concentrée et le N-ArOC-aminoacide est isolé par la voie la plus appropriée, en fonction de ses propriétés physico-chimiques.

Ce premier mode d'exécution du procédé selon l'invention est préféré lorsque l'on souhaite incorporer in fine, au sein d'un peptide, le dérivé N^{ω}-carbamoyle d'un diaminoacide. Dans ce cas particulier des α,ω-diaminoacides où il est nécessaire de bloquer sélectivement la fonction amino de la chaîne latérale, le dérivé N^{ω}-ArOC peut être préparé en recourant à des techniques bien connues d'acylation sélective, par exemple par l'intermédiaire du complexe cuivrique selon une procédure similaire à celle décrite notamment dans "Methoden Der Organischen Chemie" (HOUBEN-WEYL), 1974, Volume XV/1, p.472, au sujet de la N^{ε}-benzyloxycarbonyl-L-lysine.

Dans ce cas précis des α,ω-diaminoacides, selon la méthode de synthèse peptidique et selon la stratégie mise en oeuvre, la fonction α-NH₂ peut être bloquée si nécessaire par tout groupement protecteur usuel ou peut constituer la composante aminée à introduire dans une chaîne peptidique dont la fonction carboxyle terminale est activée de manière classique. Le N^{ω}-ArOC-aminoacide peut être couplé à cette chaîne, par exemple sous forme silylée, la silylation pouvant être réalisée au moyen notamment de chlorure de triméthylsilyle et de triéthylamine, d'hexaméthyldisilazane, de bistriméthylsilylacétamide, ou de triméthylcyanosilane (TMSCN), tel que décrit dans le brevet européen EP-B-184243. Toute autre méthode de couplage peut également être utilisée, telle que, par exemple, la mise en oeuvre du N^{ω}-ArOC-aminoacide tel quel, en combinaison avec un ester actif en milieu semi-aqueux.

De même, selon la méthode de synthèse peptidique et selon la stratégie mise en oeuvre, la fonction carboxyle de tout N-ArOC-aminoacide peut être éventuellement bloquée par un groupement protecteur usuel ou activée par un agent d'activation classique.

Après incorporation du N-ArOC-aminoacide dans un fragment peptidique, la synthèse peptidique peut être poursuivie de manière classique par condensation entre un fragment réagissant via sa fonction amino (composant aminé) et un fragment réagissant via sa fonction carboxyle (composant carboxylique). La présence du groupement N-ArOC, tant sur le composant aminé que sur le composant carboxylique, ne gêne en rien ni le couplage avec d'autres acides aminés ou fragments peptidiques ni les méthodes d'activation habituellement mises en oeuvre.

Ce premier mode d'exécution du procédé selon l'invention est particulièrement préféré pour synthétiser des peptides renfermant des restes L, D, DL 2-amino-4-uréido-butyrique (norcitrulline), citrulline et homocitrulline à partir respectivement des acides aminés précurseurs L, D ou DL correspondants, c'est-à-dire respectivement à partir de l'acide 2,4-diaminobutyrique, de l'ornithine et de la lysine. Ces acides aminés peuvent être introduits sans difficulté dans une chaîne peptidique intermédiaire, de préférence directement sous la forme de leur dérivé N^{ω}-PhOC ou N^{ω}-Toc, lesquels sont ultérieurement transformés in situ au sein du peptide ou d'un fragment peptidique en dérivé N^{ω}-carbamoyle avec un rendement très élevé et, dans le cas de structures D ou L, sans modification de la pureté chirale, élément primordial de l'activité physiologique ou thérapeutique.

Dans un second mode d'exécution du procédé d'obtention de N-carbamoyl-peptides selon l'invention, le peptide intermédiaire comportant un ou plusieurs groupements N-ArOC est synthétisé au départ d'un peptide précurseur, par introduction du groupement ArOC sur la ou les fonctions amino à carbamoyler au sein de la chaîne peptidique et non pas au niveau de l'acide aminé. Ce mode d'exécution du procédé peut être utilisé notamment pour carbamoyler une ou plusieurs fonctions amino libres d'un peptide naturel, mais peut aussi être mis en oeuvre dans le cas de peptides synthétiques. Dans ce dernier cas, le peptide intermédiaire est préparé en deux stades successifs : un premier stade consiste en la synthèse du peptide précurseur comprenant le reste d'aminoacide comportant la fonction amino à carbamoyler, au départ des différents aminoacides le constituant et un deuxième stade consiste en la transformation du peptide précurseur en peptide intermédiaire comportant un groupement N-ArOC. L'acide aminé dont on souhaite transformer une fonction amino en fonction uréido ou uréino est incorporé au sein de la chaîne peptidique du peptide précurseur de manière classique, en masquant cette fonction amino durant la synthèse peptidique par un groupement protecteur usuel, par exemple par un groupement benzyloxycarbonyle, tert-butyloxycarbonyle ou phtaloyle. A un moment ultérieur de la synthèse, après incorporation dans la chaîne peptidique du reste d'aminoacide comportant la fonction amino protégée et au moment le plus opportun en fonction de la stratégie adoptée, le groupement usuel protégeant cette fonction est éliminé de manière classique pour obtenir le peptide précurseur.

Dans le peptide précurseur, naturel ou synthétique, la fonction amino que l'on désire carbamoyler, à ce moment sous forme libre, est alors transformée, dans un second stade, en une fonction ArOC-amino pour former le peptide intermédiaire. L'introduction de ce groupement ArOC est effectuée, par réaction de cette fonction amino libre avec un agent d'aryloxycarbonylation, par exemple avec un chloroformiate d'aryle ou avec un aryloxycarbonyloxysuccinimide. Dans le cas où la fonction amino que l'on désire carbamoyler est une fonction ω-amino, l'introduction du groupement ArOC peut être réalisée classiquement par une méthode similaire à celle utilisée pour fixer sur une fonction amino un groupement protecteur classique, comme le groupement benzyloxycarbonyle. Dans le cas où la fonction amino que l'on désire carbamoyler est une fonction α-amino, une procédure d'introduction du groupement N-ArOC qui a conduit à d'excellents résultats et qui est dès lors préférée, consiste à mettre en oeuvre le peptide précurseur sous la forme de son dérivé persilylé, lequel peut être obtenu, de manière connue en soi, par réaction avec un agent de silylation envisagé plus haut. Le dérivé persilylé est mis en contact avec l'agent d'aryloxycarbonylation, de préférence dans des proportions stoechiométriques, dans un solvant immiscible à l'eau. Des solvants qui conviennent sont notamment le méthyltertiobutyléther, le dichlorométhane et l'acétate d'éthyle. Ce dernier solvant est préféré. La réaction entre le dérivé persilylé et l'agent d'aryloxycarbonylation est réalisée à une température de 0 à 40°C, de préférence à température ambiante. Après quelques minutes de réaction, de préférence pas plus de deux minutes, de l'eau est ajoutée au milieu de réaction et le système biphasique obtenu est agité pendant une durée d'environ 10 à 30 minutes. Après séparation des phases, le peptide intermédiaire comportant le groupement N-ArOC est ensuite isolé de la phase organique par la voie la plus appropriée, en fonction de ses propriétés physico-chimiques. Cette procédure préférée permet de préparer le peptide intermédiaire de manière très sélective, en respectant la chiralité des composés et en évitant la formation de produits secondaires, de nature hétérocyclique.

De préférence après purification du peptide intermédiaire comportant le ou les groupements N-ArOC, par toute méthode connue, par exemple par cristallisation, celui-ci est mis en contact avec un composé R1R2NH tel que défini plus haut, pour donner par le procédé selon l'invention, le peptide dans lequel la fonction amino libre du peptide précurseur a été transformée en fonction uréino.

Recourant à un groupement protecteur classique de la fonction amino à carbamoyler en vue de l'introduction de cet acide aminé dans la chaîne peptidique du peptide précurseur, groupement protecteur qu'il faut ensuite éliminer et remplacer par un groupement ArOC pour obtenir le peptide intermédiaire, ce second mode d'exécution du procédé selon l'invention implique, pour un peptide synthétique, un nombre d'étapes plus important que le premier mode d'exécution du procédé dans lequel le N-ArOC-peptide intermédiaire est préparé dès le départ avec un acide aminé N-ArOC. En conséquence, lorsque le peptide à carbamoyler est un peptide préparé par voie synthétique au départ des acides aminés constituants, le premier mode d'exécution du procédé d'obtention de N-carbamoyl-peptides selon l'invention est préféré. Par contre, dans le cas de peptides naturels, le second mode d'exécution du procédé d'obtention de N-carbamoyl-peptides selon l'invention est préféré.

Quel que soit le mode d'incorporation du groupement N-ArOC au sein de la structure peptidique, le reste d'aminoacide comportant un groupement N-ArOC est ensuite transformé in situ, dans la chaîne peptidique, au moment jugé le plus opportun, par réaction avec un composé R1R2NH tel que défini ci-avant pour donner, par le procédé selon l'invention, le N-carbamoyl-peptide correspondant.

Le composé R1R2NH est mis en oeuvre en excès par rapport au N-ArOC-peptide intermédiaire, généralement en quantité au moins 2 fois supérieure à la quantité stoechiométrique, le plus souvent en quantité de 5 à 100 fois, de préférence de 5 à 20 fois, supérieure à la quantité stoechiométrique. La réaction est effectuée dans tout milieu adéquat, notamment dans un solvant organique tel que le tétrahydrofurane, la diméthylformamide, l'acétate d'éthyle ou un alcool comme le méthanol. Le composé R1R2NH est mis en oeuvre soit sous forme anhydre, soit en solution aqueuse concentrée. La réaction est effectuée généralement à une température de -20 à +50 °C. De préférence, le procédé selon l'invention est effectué à une température de 0 à 20 °C, une telle température permettant d'obtenir une bonne vitesse de réaction, tout en conservant une sélectivité très élevée.

Il est évident que les éventuels autres groupements protecteurs présents dans la structure peptidique intermédiaire lors de la mise en oeuvre du procédé selon l'invention doivent être stables dans les conditions d'ammonolyse ou d'aminolyse du groupement N-ArOC, à moins que l'on ne désire profiter de l'ammonolyse ou de l'aminolyse du procédé selon l'invention pour éliminer parallèlement d'autres groupements protecteurs encore présents dans la structure peptidique.

L'invention concerne aussi les diaminoacides N^{ω}-substitués par un groupement ArOC et les peptides intermédiaires contenant un ou plusieurs restes de diaminoacides N^{ω}-substitués par un groupement ArOC. Ces diaminoacides et peptides intermédiaires peuvent être éventuellement substitués, protégés ou activés. En conséquence, l'invention concerne les composés répondant à la formule générale 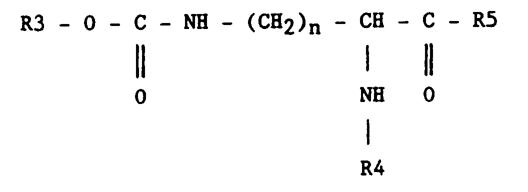 dans laquelle
- R3 représente un groupement aryle, substitué ou non par un ou plusieurs groupements alkyles comportant de 1 à 4 atomes de carbone
- R4 représente un atome d'hydrogène, un groupement protecteur de la fonction amino, un acide aminé ou un peptide dont certaines fonctions sont éventuellement substituées par un groupement protecteur ou par un groupement d'activation
- R5 représente un groupement hydroxyle, un atome d'halogène, un groupement protecteur de la fonction carboxyle, un groupement d'activation, un groupement amino, un acide aminé ou un peptide dont certaines fonctions sont éventuellement substituées par un groupement protecteur ou par un groupement d'activation
- n est un nombre entier de 1 à 10.

A titre d'exemples non limitatifs de groupements protecteurs de la fonction amino pouvant être représentés par R4, on peut citer, notamment, les groupements de type alkyle ou aralkyle, substitués ou non, comme le groupement benzyle, diphénylméthyle, di(méthoxyphényl)méthyle ou triphénylméthyle (trityle), les groupements de type acyle, substitués ou non, comme le groupement formyle, acétyle, trifluoroacétyle, benzoyle ou phtaloyle, les groupements de type aralkyloxycarbonyle, substitués ou non, comme le groupement benzyloxycarbonyle, p-chlorobenzyloxycarbonyle, p-bromobenzyloxycarbonyle, p-nitrobenzyloxycarbonyle, p-méthoxybenzyloxycarbonyle, benzhydryloxycarbonyle, 2-(p-biphénylyl)-isopropyloxycarbonyle, 2-(3,5-diméthoxyphényl)isopropyloxycarbonyle, p-phénylazobenzyloxycarbonyle, triphénylphosphonoéthyloxycarbonyle ou 9-fluorénylméthyloxycarbonyle, les groupements de type alkyloxycarbonyle, substitués ou non, comme le groupement tert-butyloxycarbonyle, tert-amyloxycarbonyle, diisopropylméthyloxycarbonyle, isopropyloxycarbonyle, éthyloxycarbonyle, allyloxycarbonyle, 2-méthylsulfonyléthyloxycarbonyle ou 2,2,2-trichloroéthyloxycarbonyle, les groupements de type cycloalkyloxycarbonyle comme le groupement cyclopentyloxycarbonyle, cyclohexyloxycarbonyle, adamantyloxycarbonyle ou isobornyloxycarbonyle, les groupements comportant un hétéroatome comme le groupement benzènesulfonyle, p-toluenesulfonyle (tosyle), mésitylènesulfonyle, méthoxytriméthylphénylsulfonyle, o-nitrophénylsulfényle ou triméthylsilane.

A titre d'exemples non limitatifs de groupements protecteurs de la fonction carboxyle pouvant être représentés par R5, on peut citer, notamment, les groupements méthyle, éthyle, t-butyle, cyclohexyle, benzyle, p-nitrobenzyle, phénacyle, triméthylsilyle, carboamidométhyle et fluorénylméthyle.

A titre d'exemples non limitatifs de groupements d'activation pouvant être représentés par R5, on peut citer, notamment, les groupements O-succinimidoyle, O-norbornènedicarboximidoyle, O-benzotriazolyle, O-oxodihydrobenzotriazolyle, O-hydroxypyridyle, pentachlorophényle, trichlorophényle, pentafluorophényle, isobutyloxycarbonyle et triméthylacétyle.

Généralement, R3 est un groupement contenant au maximum 12 atomes de carbone. Le plus souvent R3 est un groupement phényle, naphtyle, tolyle, xylyle, mésitylyle, éthylphényle, diéthylphényle, propylphényle ou isopropylphényle. De préférence, R3 est un groupement phényle ou tolyle. De manière particulièrement préférée, R3 est un groupement phényle ou p-tolyle.

De préférence, n est un nombre entier de 2 à 6. De manière particulièrement préférée, n est un nombre entier de 2 à 4.

Enfin, des composés ayant conduit à de très bons résultats dans le procédé selon l'invention sont notamment ceux où :
- R3 représente un groupement phényle
- R4 représente un atome d'hydrogène ou le dipeptide Z-Ser-Tyr-
- R5 représente un groupement hydroxyle, un acide aminé ou un peptide dont certaines fonctions sont éventuellement substituées par un groupement protecteur ou par un groupement d'activation.
- n est égal à 2, 3 ou 4.

Ces composés préférés selon l'invention sont des dérivés N^{ω}-PhOC de l'acide 2,4-diaminobutyrique, de l'ornithine ou de la lysine et des peptides ou structures peptidiques contenant un dérivé N^{ω}-PhOC d'un reste d'un de ces diaminoacides, quelle que soit leur configuration (D, L ou DL).

A titre d'exemples, on peut citer, comme composés selon l'invention, l'acide N^{γ}-PhOC-diaminobutyrique et les peptides comprenant un reste diaminobutyrique en position quelconque dont la fonction γ-amino est substituée par un groupement PhOC ; la N^{δ}-PhOC-D-ornithine et les peptides comprenant un reste D-ornithine en position quelconque dont la fonction δ-amino est substituée par un groupement PhOC ; la N^{ε}-PhOC-lysine et les peptides comprenant un reste lysine en position quelconque dont la fonction ε-amino est substituée par un groupement PhOC. Un peptide contenant un reste de diaminoacide N^{ω}-substitué par un groupement ArOC est, par exemple, le tripeptide Z-Ser-Tyr-D-Orn(PhOC).

Ces composés selon l'invention sont d'excellents intermédiaires de synthèse pour la préparation de peptides divers incluant des restes d'aminoacides comportant un groupement uréino tels que les restes citrulline, homocitrulline, 2-amino-4-uréido-butyrique : les acides aminés N^{ω}-ArOC peuvent être aisément incorporés au sein de structures peptidiques intermédiaires, lesquelles peuvent ensuite être aisément transformées en N^{ω}-carbamoyl-peptides par le procédé selon l'invention.

En particulier, ces composés sont d'excellents intermédiaires pour la fabrication en phase liquide de fragments d'hormones modifiées.

L'invention concerne aussi les peptides de formule générale
R6-Ser(R7)-Tyr(R8)-R9-R10
dans laquelle
- R6 est un atome d'hydrogène ou un groupement protecteur de la fonction amino,
- Ser(R7) est un reste sérine avec R7 représentant un atome d'hydrogène ou un groupement protecteur de la fonction hydroxyle de la chaîne latérale du reste sérine
- Tyr(R8) est un reste tyrosine avec R8 représentant un atome d'hydrogène ou un groupement protecteur pour la fonction hydroxyle de la chaîne latérale du reste tyrosine
- R9 est, dans une première variante, un reste 2,4-diamino-butyrique ou ornithine de configuration D, L ou DL et dans une deuxième variante, un reste 2-amino-4-uréido-butyrique, citrulline ou homocitrulline de configuration D, L ou DL, dont les atomes d'hydrogène de la fonction carbamoyle sont éventuellement substitués par un groupement alkyle, cycloalkyle ou aralkyle contenant au maximum 12 atomes de carbone ou par un groupement cycloalkylène contenant de 3 à 6 atomes de carbone.
- R10 est un radical hydroxyle, un acide aminé ou un peptide dont certaines fonctions sont éventuellement substituées par un groupement protecteur.

A titre d'exemples non limitatifs de groupements protecteurs de la fonction amino pouvant être représentés par R6, on peut citer notamment les groupements benzyloxycarbonyle et tert-butyloxycarbonyle.

A titre d'exemples non limitatifs de groupements protecteurs de la fonction hydroxyle de la chaîne latérale du reste sérine ou du reste tyrosine pouvant être représentés par R7 ou par R8, on peut citer notamment les groupements benzyle, benzyloxycarbonyle, triméthylsilyle.

De préférence, R9 représente des restes de configuration D. De préférence, R10 est un radical hydroxyle ou un fragment peptidique comportant de 1 à 5 acides aminés. De manière particulièrement préférée, R10 est un radical hydroxyle.

Lorsque R9 est un reste d'aminoacide selon la première variante, les peptides sont notamment des précurseurs de certains peptides intermédiaires comportant un groupement N-aryloxycarbonyle mis en oeuvre dans le procédé selon l'invention. La préparation de ces peptides précurseurs peut être effectuée par toute méthode classique de synthèse peptidique, soit en phase "solide" selon la technique dite de Merrifield, soit en phase liquide.

Lorsque R9 est un reste d'aminoacide selon la deuxième variante, les peptides sont notamment des fragments de certaines hormones modifiées. La préparation de ces fragments peptidiques peut être aisément effectuée en phase liquide grâce au procédé selon l'invention, en mettant en oeuvre leurs intermédiaires N^{ω}-ArOC selon l'invention, éventuellement obtenus au départ des peptides précurseurs ci-dessus.

Les représentations symboliques des aminoacides et des peptides adoptées dans la description et les exemples suivent les recommandations IUPAC de nomenclature, généralement adoptées et décrites par exemple dans "Nomenclature and Symbolism for Amino Acids and Peptides, Recommendations 1983", Eur. J. Biochem. (1984), 138, p.9-37. Par convention, les peptides sont représentés avec leur extrémité N-terminale à gauche et leur extrémité C-terminale à droite. Les abréviations utilisées pour désigner les restes d'acides aminés dérivent des noms triviaux des acides aminés et sont A₂bu, acide 2,4-diaminobutyrique; Orn, ornithine; Lys, lysine; Nci, acide 2-amino-4-uréido-butyrique ou norcitrulline; Cit, citrulline; Hci, homocitrulline, Ser, sérine; Tyr, tyrosine; Leu, Leucine; Arg, Arginine; Pro, proline; Gly, glycine; Ala, alanine. Sauf stipulation contraire, tous les acides aminés décrits sont les acides aminés sous forme L.

Les différents produits et intermédiaires de synthèse rapportés dans les exemples ont été caractérisés par différentes méthodes analytiques, mises en oeuvre dans les conditions suivantes :
- Chromatographie en couche mince (CCM) :
   . Plaques de silicagel MERCK 60F-254
   . éluants :
      Rf(1) HCOOMe:HCOOH:MeOH 95:2,5:2,5
      Rf(2) EtOAc:EtOH:HOAc 8:1:1
      Rf(3) CH₃CN:CHCl₃:HOAc:H₂O 7:7:4:2
      Rf(4) HCOOMe:HCOOH:MeOH:H₂O 92,5:2,5:2,5:2,5
- Chromatographie HPLC :
   . Colonne C-18 Vydac 5µ
   . Elution : gradient de 98 % A + 2% B jusqu'à 25 % A + 75 % B en 49 minutes (A = H₂O 0,1 % en TFA ; B = CH₃CN 0,1 % en TFA)
   . Débit = 2 ml/min
   . Détection : UV 220 nm.
- Résonance magnétique nucléaire (RMN) :
   . Appareil Brüker AMX 500 MHz
   . Shift donnés en ppm
   . Allures des résonances : m=multiplet, s=singulet, d=doublet,
      t=triplet, q=quadruplet quint=quintuplet.

Les abréviations suivantes sont utilisées dans les exemples ci-après :
α = rotation optique, mesurée à 589 nm à 25 °C
CH₃CN = acétonitrile
DMF = diméthylformamide
EtOAc = acétate d'éthyle
EtOH = alcool éthylique
HCOOH = acide formique
HCOOMe = formiate de méthyle
HOAc = acide acétique
i.BuOCOCl = chloroformiate d'isobutyle
MeOH = alcool méthylique
MTBE = méthyltertiobutyléther
NMM = N-méthylmorpholine
NMP = N-méthylpyrrolidone
PhEt = phényléthyle
PhOC = phényloxycarbonyle
PhOC-OSu = phényloxycarbonyloxysuccinimide
Pht = phtaloyle
PivCl = chlorure de pivaloyle
PF = point de fusion
Pyr = pyridine
TEA = triéthylamine
TFA = acide trifluoroacétique
THF = tétrahydrofurane
TMS = triméthylsilyle
TMSCN = triméthylcyanosilane
Z = benzyloxycarbonyle

### Exemple 1 : Préparation de N^{ε}-PhOC-lysine à partir de lysine

Une solution contenant 36,5 g LysHCl et 16 g de NaOH dans 200 ml d'eau est mélangée avec 25 g de CuSO₄.5H₂O dissous dans 80 ml d'eau 25 g de NaHCO₃ sont ensuite introduits sous agitation.

Après dissolution du bicarbonate, la solution est refroidie à 0 °C, puis traitée goutte-à-goutte avec 30 ml de chloroformiate de phényle (durée de l'introduction 1h). Après un mûrissage de 3 h à température ambiante, le précipité formé est filtré, lavé 2 fois avec 200 ml d'eau, 2 fois avec 200 ml d'acétone puis séché à l'air. Le complexe cuivrique ainsi obtenu est suspendu dans 400 ml d'eau puis décomposé par addition de 80 ml de HCl concentré. Les ions cuivriques sont ensuite précipités par addition, en petites portions, de la quantité stoechiométrique de sulfure de sodium. Après dégazage pour éliminer le H₂S qui s'est éventuellement formé, la suspension est mélangée avec 10 g de célite.

Après filtration et lavage du gâteau par 200 ml d'eau, le pH du filtrat est ajusté à 7 par introduction de NaHCO₃. La N^{ε}-PhOC-Lys précipite. Après 1 h à 0 °C, elle est collectée par filtration, lavée à l'eau, au méthanol et à l'acétone et puis séchée.
Rendement : 45 g (84 %)
PF : 211-215 °C (décomp.)
α : +16,6 (c = 1/1 N HCl)
CCM : Rf(3)= 0,50
HPLC : tR = 11,70 min
RMN(H-1) : (réf DMSO-d6 à 2,49ppm ; produit dissous dans le DMSO-d6 en mettant une goutte de TFA)
8,27 (3H s large) NH3 7,70 (1H s large) NH
7,35 (2H t) m.phényl 7,18 (1H t) p.phényl
7,08 (2H d) o.phényl 3,89 (1H s large) Hα
3,06 (2H m) Hε
1,79 (2H m), 1,48 (3H m) et 1,37 (1Hm ) Hβ,Hγ et Hδ.

### Exemple 2 : Préparation de N^{δ}-PhOC-D-ornithine à partir de D-ornithine

La N^{δ}-PhOC-D-Ornithine a été préparée, avec un rendement identique, suivant la même recette que la N^{ε}-PhOC-lysine de l'exemple 1.
PF = 197-199 °C (décomp.)
α = -17,8 (c = 1,1 /1 N HCl)
CCM : Rf(3)= 0,54
HPLC : tR = 9,69 min.

### Exemple 3 : Préparation de l'acide N^{γ}-PhOC-D-diaimobutyrique à partir de l'acide D-diaminobutyrique

L'acide N^{γ}-PhOC-D-diaminobutyrique a été préparé, avec un rendement de 65 %, suivant la même recette que la N^{ε}-PhOC-lysine de l'exemple 1.
PF = 202-204 °C
CCM : Rf(3)= 0,60
HPLC : tR = 7,55 min.

### Exemple 4 : Synthèse de Z-Ser-Tyr-D-Orn(PhOC)

4,03 g (10 mmol) Z-Ser-Tyr sont dissous dans 15 ml de THF puis neutralisés par 1,10 ml (10 mmol) de NMM. L'activation du peptide est effectuée à -15 °C par addition de 1,3 ml (10 mmol) de i.BuOCOCl. Après 3 minutes, on procède au couplage par addition d'une solution refroidie à 0 °C de N^{δ}-PhOC-D-Ornithine persilylée, préparée à partir d'une suspension de 3,15 g (12,5 mmol) de N^{δ}-PhOC-D-Ornithine dans 6,5 ml (50 mmol) de TMSCN que l'on porte à reflux jusqu'à l'obtention d'une solution limpide.

Après 5 minutes de réaction à basse température, on remonte la température à 20 °C et on laisse mûrir pendant 1/2 h. La réaction est interrompue par addition de 60 ml d'EtOAc et 50 ml d'une solution aqueuse de KHSO₄ à 5 %. Après élimination de la phase aqueuse, la phase organique est lavée avec 20 ml d'eau, puis concentrée à sec. Le résidu est dissous dans 60 ml de méthanol et la cristallisation du tripeptide sous forme de sel d'ammonium induite par addition d'ammoniaque concentré jusqu'à pH 8.
Rendement : 5,1 g (78%)
Données analytiques d'un échantillon libéré de son sel:
PF : 140°-160 °C (décomp.)
α : -23,3 (c = 1,06 / MeOH)
CCM : Rf(1) = 0,76 / Rf(2) : 0,70
HPLC : tR = 23,43 min
RMN(H-1) (réf. CD₃OD à 3,31) :
7,30 (7H m) 5H du Z, 2H du PhOC 7,19 (1H t) p.PhOC
7,08 (4H m) 2H du PhOC, 2H de Tyr 6,71 (2H d) 2H de Tyr
5,10 (2H q) phénylCH₂O 4,67 (1H q) TyrHα
4,41 (1H q) OrnHα 4,17(1H t) SerHα
3,79 (2H m) Serβ's 3,16(2H t) Ornδ's
3,10 (1H q) TyrβA 2,90 (1H q) TyrβB
1,89 (1H m) 1,74 (1H m) 1,50 (2H m) OrnHβ's + OrnHγ's.

### Exemple 5 : Synthèse de Z-Ser-Tyr-D-Cit

2,0 g (3,15 mmol) de Z-Ser-Tyr-D-Orn(PhOC) obtenus dans l'exemple 4 sont ajoutés à un mélange de 20 ml de MeOH et 20 ml de NH₄OH à 25 %. La solution obtenue est conservée à 40 °C pendant 75 minutes, puis évaporée à sec. Le résidu est remis en suspension dans un mélange de 10 ml d'eau et de 10 ml d'EtOAc. La cristallisation du tripeptide Z-Ser-Tyr-Cit est induite par acidification de la phase aqueuse (pH 3) à l'aide d'une solution de KHSO₄ concentrée. Le peptide est collecté par filtration, puis séché. Rendement : 1,45 g (82 %). Les données analytiques du tripeptide Z-Ser-Tyr-D-Cit produit sont :
PF : 132 - 138°C (décomp.)
α : - 8,10 (c=1,1 /DMF)
CCM : Rf(4) = 0,17
HPLC : tR = 16,44 min
RMN (H-1) :
   Spectre identique à celui de Z-Ser-Tyr-D-Orn(PhOC) (exemple 4), mais disparition des résonances du groupement PhOC et apparition des résonances du groupe carbamoyle :
5,90 (1H t) NH 5,36 (2H s) NH₂

### Exemple 6 : Synthèse de Z-Ser-Tyr-D-Cit(PhEt)

654 mg (1 mmol) de Z-Ser-Tyr-D-Orn(PhOC) cristallisé sous forme de son sel d'ammonium est suspendu dans un système biphasique composé de 10 ml de KHSO₄ à 2 % et de 20 ml d'EtOAc. Le mélange est agité à 50 °C pendant 1 heure. La phase organique est recueillie, concentrée à moitié, puis traitée avec 1,22 g (10 mmol) de phényléthylamine. Le cours de la réaction peut être suivi par CCM. Dès la conversion complète du tripeptide, le milieu réactionnel est concentré à sec et le solide obtenu lavé deux fois par 20 ml de MTBE. On recueille ainsi le tripeptide sous forme de sel de phényléthylamine (Z-Ser-Tyr-D-Cit(PhEt)-NHPhEt). Pour obtenir le tripeptide libre, le sel est dissous dans un minimum de méthanol, puis précipité dans un grand volume d'une solution de KHSO₄ à 5 %. Après filtration et lavage à l'eau, on obtient le tripeptide Z-Ser-Tyr-D-Cit(PhEt).
Rdt : 630 mg (95 %)
PF : 160-169 °C (décomp.)
α : -21,7 (c = 1 / MeOH)
CCM : Rf(1) = 0,65 / Rf(2) = 0,51
HPLC : tR = 23,59 min
RMN(H-1) (réf DMSO-d6 à 2,49ppm) :
9,12 (1H s) OHTyr 8,16 (1H d) NHOrn 7,91 (1h d) NHTyr
7,35 (5H m) H's Z 7,28 (2H m) + 7,17 (3H m) H's de Ph.éthylamine
7,21 (1H d) NHSer 6,99 (2H d) Hδ'sTyr 6,60 (2H d) Hε'sTyr
5,84 (1H t) NH uréido-Orn
5,77 (1H t) NH uréido-Phényléthylamine
5,01 (2H q) CH₂ Z 4,48 (1H m) HαTyr 4,13 (1H m) HαOrn
4,05 (1H m) HαSer 3,48 (2H d) Hβ'sSer
3,19 (2H q) NHCH₂ Phényléthylamine 2,92 (2H q) Hδ'sOrn
2,87 (1H q) HβATyr 2,69 (1H q) HβBTyr
2,64 (2H t) CH₂Ph. Phényléthylamine 1,63 (1H m) HβAOrn
1,50 (1H m) HβBOrn 1,28 (2H m) Hγ'sOrn

### Exemple 7 : Synthèse de Z-Ser-Tyr-D-Cit(2-méthylbutyl)

La synthèse de l'exemple 6 a été répétée en utilisant la 2-méthylbutylamine à la place de phényléthylamine. La Z-Ser-Tyr-D-Cit(2-méthylbutyl) a été obtenue.
CCM : Rf(1) = 0,63 / Rf(2) = 0,52
HPLC : tR = 23,07 min
RMN(H-1) : Par rapport au produit de départ (exemple 4), les raies du groupement PhOC ont disparues et apparaissent des raies du résidu 2-Méthylbutylamine à 3,03 et 2,90 pour le N-CH₂-; à 1,40 pour le N-CH₂-CH(CH₃)-CH₂-; à 1,12 pour le N-CH₂-CH-, à 0,89 pour le N-CH₂-CH(CH₃)-CH₂-CH₃ et à 0,87 pour le N-CH₂-CH(CH₃)-

### Exemple 8 : Synthèse de Z-Ser-Tyr-D-Cit(tétraméthylène)

La synthèse de l'exemple 6 a été répétée en utilisant la pyrrolidine à la place de phényléthylamine. La Z-Ser-Tyr-D-Cit-(tétraméthylène) a été obtenue.
CCM : Rf(1) = 0,35 / Rf(2) = 0,21
HPLC : tR = 19,18 min
RMN(H-1) : les raies dues au résidu pyrrolidine sont situées à 3,30 (4H) et à 1,87 (4H)

### Exemple 9 : Synthèse de Z-Ser-Tyr-D-Orn(PhOC) à partir de Z-Ser-Tyr-D-Orn

### a) Synthèse de Z-Ser-Tyr-D-Orn(Pht)

Le tripeptide Z-Ser-Tyr-D-Orn(Pht) est d'abord préparé par réaction de Z-Ser-Tyr avec δ-Pht-D-ornithine selon un mode opératoire similaire à celui de l'exemple 4. Le tripeptide recherché est obtenu sous forme de sel d'ammonium, avec un rendement de 85 %.
Données analytiques :
HPLC : tR = 24,06 min
RMN (H-1) (dans le DMSO-d6) :
7,80 (4H, m) H'sPht 7,31 (5H, m) H's Z 6,96 (2H, d) HδTyr
6,60 (2H, d) HεTyr 4,96 (2H, q) CH₂-Z 4,41 (1H m) HαTyr
4,15 (1H ,m) HαOrn 3,99 (1H, m) HαSer 3,53 (2H, m) HδOrn
3,44 (2H, d) HβSer 2,83 (1H dd) HβATyr 2,67 (1H dd) HβBTyr
1,65 (1H m) + 1,52 (3H m) Hβ + HγOrn

### b) Obtention de Z-Ser-Tyr-D-Orn

La fonction latérale du reste ornithine est déprotégée par hydrazinolyse : 28 g de Z-Ser-Tyr-D-Orn(Pht) sont mis en suspension dans 1,3 L de MeOH. Après addition de 10 ml de NH₂NH₂.H₂O, le mélange est chauffé jusqu'à ébullition. Après 2 heures, le produit de réaction commence à cristalliser. Une heure plus tard, le chauffage est arrêté et le mélange refroidi à température ambiente. Le précipité est collecté par filtration. Après lavage à l'eau et au méthanol froid, le gâteau est séché pour fournir 90 % de Z-Ser-Tyr-D-Orn pur.
HPLC : tR = 15,37 min.
RMN(H-1) (dans le CD₃OD) :
7,34 (5H m) H-arom. Z 7,08 (2H d) HδTyr 6,73 (2H m) HεTyr
5,09 (1H d) et 4,98 (1H d) CH₂-Z 4,59 (1H m) HαTyr
4,45 (1H m) HαOrn 4,13 (1H m) HαSer 3,68 (2H m) HβSer
3,13 (1H dd) HβATyr 2,90 (3H m) HβBTyr + HδOrn
1,94 (1H m), 1,77 (1H m) et 1,63 (2H m) Hβ + HγOrn

### c) Synthèse de Z-Ser-Tyr-D-Orn(PhOC)

Le groupement PhOC est introduit sur la chaîne latérale du reste ornithine de la manière suivante :
5,17 g (10 mmol) de Z-Ser-Tyr-D-Orn sont dissous dans 50 ml d'un mélange THF:H₂O 1:1 en volume. Puis le pH est ajusté à 7,5 par addition de TEA. Ensuite, 3,54 g (15 mmol) de PhOC-OSu sont introduits dans le réacteur. La conversion est complète après un temps de mûrissage de 2 h 30. Le THF est éliminé sous vide, puis sont ajoutés 100 ml d'EtOAc et, sous agitation, 25 ml d'une solution aqueuse de KHSO₄ à 10 %. La phase organique décantée est lavée avec 50 ml d'eau, puis concentrée à sec. Le résidu est repris par 50 ml de MeOH et le pH ajusté à 8 à l'aide d'ammoniaque concentré. Le tripeptide Z-Ser-Tyr-D-Orn(PhOC) cristallise sous forme de sel d'ammonium. Il est identique au produit synthétisé via la voie directe décrite à l'exemple 4.

### Exemple 10 : Synthèse de NH₂-CO-Ala-Phe

### a) Synthèse de PhOC-Ala

20 mmol d'alanine sont traitées à reflux avec 30 mmol de TMSCN jusqu'à solubilisation complète. Après dilution avec 30 ml de dichlorométhane, le mélange est refroidi à -15 °C. Ensuite sont ajoutées lentement 20 mmol de chloroformiate de phényle. Après 10 minutes, la solution est concentrée à sec. Le résidu est repris par 40 ml de dichlorométhane et lavé par 50 ml d'une solution aqueuse d'acide citrique à 5 %, puis par 50 ml d'eau. La phase organique est concentrée, reprise par 40 ml d'éther sulfurique chaud, puis diluée à l'hexane jusqu'à apparition d'un trouble. Après une nuit au frigo, les cristaux formés sont filtrés, lavés et séchés pour obtenir 3,6 g de PhOC-Ala (Rdt = 86 %).
PF : 119-124°C
α : -45,6 (c = 1 / MeOH)
HPLC : tR = 13,45 min
RMN(H-1) : (Dans le CDCl₃ ; pour certains protons deux formes apparaissent Majeur=M mineur=m) :
7,36 (2H t) PhOC méta 7,21 (1H t) PhOC para 7,14 ( 2H d) PhOC ortho
6,51 (d) NH-m et 5,62 (d) NH-M 4,49 (1H quint) Hα
1,60 (d) CH₃-m 1,55 (d) CH₃-M.

### b) Synthèse de PhOC-Ala-Phe

2,1 g (10 mmol) de PhOC-Ala, dissous dans 10 ml de dichlorométhane sont neutralisés avec 1,1 ml (10 mmol) de NMM. La solution refroidie à -10 °C est traitée avec 1,3 ml (10 mmol) d'i.BuOCOCl. Après 4 minutes d'activation à -10 °C, la solution de phénylalanine persilylée est ajoutée et après 1 heure de mûrissage à température ambiante, le dipeptide formé est désilylé par addition de 0,2 ml d'eau. Puis le mélange est lavé par 100 ml d'une solution aqueuse de KHSO₄ à 3 %. Le PhOC-Ala-Phe cristallise dans le système biphasique. Après une nuit au frigo, il est récupéré par filtration. Rdt : 3,1 g (86 %)
PF : 143-144 °C
α : -19,5 (c = 1 / MeOH)
HPLC : tR= 22,63 min
RMN (H-1) (dans le CDCl₃)
7,3 à 7,1 (10H m) H aromat. PhOC + Phe 6,74 (1H d) NH Phe
6,12 (1H d) NH Ala 4,77 (1H q) HαPhe 4,23 ( 1H quint) HαAla
3,20 (1H dd) HβAPhe 3,06 (1H dd) HβBPhe

### c) conversion en NH₂-CO-Ala-Phe

30 mg de PhOC-Ala-Phe sont dissous dans 0,5 ml d'un mélange 1:1 en volume d'ammoniaque concentré (25 %) et de MeOH. Après 3 heures, l'analyse HPLC indique une conversion complète et sélective (produits secondaires < 2 %). Le milieu réactionnel est dilué au THF jusqu'à apparition d'un trouble. Après une nuit au frigo, la quantité théorique de NH₂-CO-Ala-Phe est récupérée sous forme de son sel d'ammonium par filtration.
α : 33,1 (c=0,4 / H₂O)
HPLC: tR = 10,14 min
RMN (H-1) dans le DMSO-d6 :
7,48 (1H d) NHPhe 7,13 (5H m) H arom.Phe 6,33 (1H d ) NHAla
5,62 (2H s) NH₂-CO 4,08 (1H q) HαPhe 3,99 (1H quint) HαAla
3,07 (1H dd) HβAPhe 2,89 (1H dd) HβBPhe 1,10 (3H d) CH₃Ala.

### Exemple 11 : Synthèse de PhOC-Phe-Val

A 2,04 g (5 mmol) de Phe-Val persilylé (TMS-Phe-Val-OTMS) est ajoutée une solution de 20 ml d'EtOAc contenant 1,17 g (5 mmol) de PhOC-OSu. Après 2 minutes sont ajoutés 15 ml d'eau. Le système biphasique est agité pendant 15 minutes, puis la phase aqueuse est éliminée. La phase organique est extraite à deux reprises avec 10 ml d'eau. Le résidu de la phase organique après évaporation est constitué de 1,6 g de PhOC-Phe-Val, contaminé par des traces de Phe-Val. En reprenant ce résidu dans un système biphasique eau / MTBE, le dipeptide libre est extrait dans la phase aqueuse et le résidu de la phase organique est du PhOC-Phe-Val de pureté peptidique supérieure à 95 %.
CCM : Rf(2) = 0,86
HPLC : tR = 25,2 min
RMN (H-1) (réf.CD₃OD à 3,30 ppm)
7,30 (7H m) 5H Phe, 2H m.PhOC 7,17 (1H t) p.PhOC
6,98 (2H d) o.PhOC 4,56 (1H t) Hα Phe
4,39 (1H d) Hα Val 3,22 (1H dd) Hβ-1 Phe
2,92 (1H dd) Hβ-2 Phe 2,20 (1H m) Hβ Val
1,00 (6H m) 2 x CH₃ Val.

## Revendications

1. Procédé d'obtention d'un peptide comportant une ou plusieurs fonctions N-carbamoyle, selon lequel on fait réagir un peptide intermédiaire, comportant un ou plusieurs groupements aryloxycarbonyle constitués par un groupement de formule générale 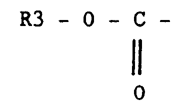 fixé sur l'atome d'azote d'une fonction amino et dans laquelle R3 représente un groupement aryle, substitué ou non par un ou plusieurs groupements alkyles comportant de 1 à 4 atomes de carbone, avec un composé de formule générale R1R2NH dans laquelle R1 et R2 représentent, indépendamment l'un de l'autre, des atomes d'hydrogène, des radicaux alkyles, cycloalkyles ou aralkyles, contenant au maximum 12 atomes de carbone, ou dans laquelle R1 et R2 forment ensemble un radical alicyclique contenant de 3 à 6 atomes de carbone.

2. Procédé selon la revendication 1, caractérisé en ce que R3 est un groupement phényle, naphtyle, tolyle, xylyle, mésitylyle, éthylphényle, diéthylphényle, propylphényle ou isopropylphényle.

3. Procédé selon la revendication 1 caractérisé en ce que R3 est un groupement phényle ou p-tolyle.

4. Procédé selon une quelconque des revendications 1 à 3, caractérisé en ce que le peptide intermédiaire comporte un groupement aryloxycarbonyle sur une ou plusieurs fonctions ω-amino.

5. Procédé selon une quelconque des revendications 1 à 4, caractérisé en ce que le peptide intermédiaire est synthétisé au départ du dérivé N-aryloxycarbonyle d'un aminoacide.

6. Procédé selon la revendication 5, caractérisé en ce que le peptide intermédiaire est synthétisé au départ du dérivé N^{ω}-aryloxycarbonyle d'un diaminoacide.

7. Procédé selon une quelconque des revendications 1 à 4, caractérisé en ce que le peptide intermédiaire est synthétisé au départ d'un peptide précurseur, par introduction du groupement aryloxycarbonyle sur la ou les fonctions amino à carbamoyler au sein de la chaîne peptidique.

8. Composés de formule générale 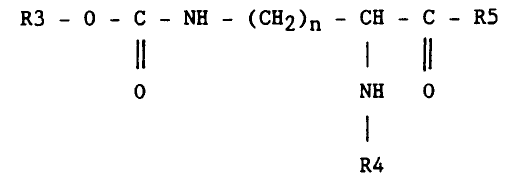 dans laquelle
- R3 représente un groupement aryle, substitué ou non par un ou plusieurs groupements alkyle comportant de 1 à 4 atomes de carbone
- R4 représente un atome d'hydrogène, un groupement protecteur de la fonction amino, un acide aminé ou un peptide dont certaines fonctions sont éventuellement substituées par un groupement protecteur ou par un groupement d'activation
- R5 représente un groupement hydroxyle, un atome d'halogène, un groupement protecteur de la fonction carboxyle, un groupement d'activation, un groupement amino, un acide aminé ou un peptide dont certaines fonctions sont éventuellement substituées par un groupement protecteur ou par un groupement d'activation
- n est un nombre entier de 1 à 10.

9. Composés selon la revendication 8, caractérisés en ce que R3 est un groupement phényle ou p-tolyle et n est un nombre entier de 2 à 4

10. Peptides de formule générale
R6-Ser(R7)-Tyr(R8)-R9-R10
dans laquelle
- R6 est un atome d'hydrogène ou un groupement protecteur de la fonction amino,
- Ser(R7) est un reste sérine avec R7 représentant un atome d'hydrogène ou un groupement protecteur de la fonction hydroxyle de la chaîne latérale du reste sérine
- Tyr(R8) est un reste tyrosine avec R8 représentant un atome d'hydrogène ou un groupement protecteur pour la fonction hydroxyle de la chaîne latérale du reste tyrosine
- R9 est, dans une première variante, un reste 2,4-diamino-butyrique ou ornithine de configuration D, L ou DL et dans une deuxième variante, un reste 2-amino-4-uréido-butyrique, citrulline ou homocitrulline de configuration D, L ou DL, dont les atomes d'hydrogène de la fonction carbamoyle sont éventuellement substitués par un groupement alkyle, cycloalkyle ou aralkyle contenant au maximum 12 atomes de carbone ou par un groupement cycloalkylène contenant de 3 à 6 atomes de carbone.
- R10 est un radical hydroxyle, un acide aminé ou un peptide dont certaines fonctions sont éventuellement substituées par un groupement protecteur.

## Claims

1. Method of producing a peptide containing one or more N-carbamoyl functional groups, according to which an intermediate peptide containing one or more aryloxycarbonyl groups consisting of a group of general formula 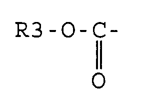 attached to the nitrogen atom of an amino functional group and in which R3 represents an aryl group, unsubstituted or substituted by one or more alkyl groups containing 1 to 4 carbon atoms, is reacted with a compound of general formula R1R2NH in which R1 and R2 represent, independently of each other, hydrogen atoms, alkyl, cycloalkyl or aralkyl radicals, containing at most 12 carbon atoms, or in which R1 and R2 together form an alicyclic radical containing 3 to 6 carbon atoms.

2. Method according to Claim 1, characterised in that R3 is a phenyl, naphthyl, tolyl, xylyl, mesitylyl, ethylphenyl, diethylphenyl, propylphenyl or isopropylphenyl group.

3. Method according to Claim 1, characterised in that R3 is a phenyl or p-tolyl group.

4. Method according to any one of Claims 1 to 3, characterised in that the intermediate peptide bears an aryloxycarbonyl group on one or more ω-amino functional groups.

5. Method according to any one of Claims 1 to 4, characterised in that the intermediate peptide is synthesised starting with the N-aryloxycarbonyl derivative of an amino acid.

6. Method according to Claim 5, characterised in that the intermediate peptide is synthesised starting with the N^{ω}-aryloxycarbonyl derivative of a diamino acid.

7. Method according to any one of Claims 1 to 4, characterised in that the intermediate peptide is synthesised starting with a precursor peptide, by introducing the aryloxycarbonyl group onto the amino functional group(s) to be carbamoylated inside the peptide chain.

8. Compounds of general formula 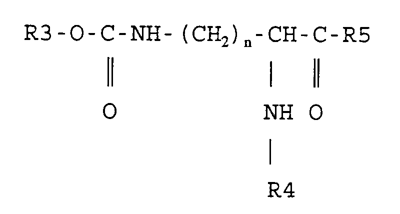 in which
- R3 represents an aryl group which is unsubstituted or substituted by one or more alkyl groups containing 1 to 4 carbon atoms
- R4 represents a hydrogen atom, a group for protecting the amino functional group, an amino acid or a peptide some of whose functional groups are optionally substituted by a protecting group or by an activating group
- R5 represents a hydroxyl group, a halogen atom, a group for protecting the carboxyl functional group, an activating group, an amino group, an amino acid or a peptide some of whose functional groups are optionally substituted by a protecting group or by an activating group
- n is an integer from 1 to 10.

9. Compound according to Claim 8, characterised in that R3 is a phenyl or p-tolyl group and n is an integer from 2 to 4.

10. Peptides of the general formula
R6-Ser(R7)-Tyr(R8)-R9-R10
in which
- R6 is a hydrogen atom or a group for protecting the amino functional group,
- Ser(R7) is a serine residue with R7 representing a hydrogen atom or a group for protecting the hydroxyl functional group of the side chain of the serine residue
- Tyr(R8) is a tyrosine residue with R8 representing a hydrogen atom or a group for protecting the hydroxyl functional group of the side chain of the tyrosine residue
- R9 is, in a first variant, a 2,4-diaminobutyric or ornithine residue of the D, L or DL configuration, and in a second variant, a 2-amino-4-ureidobutyric, citrulline or homocitrulline residue of the D, L or DL configuration, in which the hydrogen atoms of the carbamoyl functional group are optionally substituted by an alkyl, cycloalkyl or aralkyl group containing at most 12 carbon atoms, or by a cycloalkylene group containing 3 to 6 carbon atoms.
- R10 is a hydroxyl radical, an amino acid or a peptide some of whose functional groups are optionally substituted by a protecting group.

## Patentansprüche

1. Verfahren zur Herstellung eines Peptids, das eine oder mehrere N-Carbamoylfunktionen umfaßt, wonach man ein Zwischenpeptid, das ein oder mehrere Aryloxycarbonylgruppen umfaßt, bestehend aus einer Gruppe der allgemeinen Formel 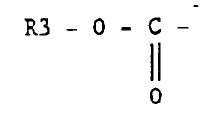 die an das Stickstoffatom einer Aminofunktion gebunden ist und worin R3 eine Arylgruppe darstellt, die substituiert oder nicht substituiert ist mit einer oder mehreren Alkylgruppen, die 1 bis 4 Kohlenstoffatome umfassen, mit einer Verbindung der allgemeinen Formel R1R2NH umsetzt, worin R1 und R2 unabhängig voneinander Wasserstoffatome, Alkyl-, Cycloalkyl- oder Aralkylreste darstellen, die maximal 12 Kohlenstoffatome enthalten, oder worin R1 und R2 zusammen einen alicyclischen Rest bilden, der 3 bis 6 Kohlenstoffatome enthält.

2. Verfahren nach Anspruch 1, dadurch gekennzeichnet, daß R3 eine Phenyl-, Naphthyl-, Tolyl-, Xylyl-, Mesitylyl-, Ethylphenyl-, Diethylphenyl-, Propylphenyl- oder Isopropylphenylgruppe ist.

3. Verfahren nach Anspruch 1, dadurch gekennzeichnet, daß R3 eine Phenyl- oder p-Tolylgruppe ist.

4. Verfahren nach einem der Ansprüche 1 bis 3, dadurch gekennzeichnet, daß das Zwischenpeptid eine Aryloxycarbonylgruppe an einer oder mehreren ω-Aminofunktionen umfaßt.

5. Verfahren nach einem der Ansprüche 1 bis 4, dadurch gekennzeichnet, daß das Zwischenpeptid zu Beginn aus einem N-Aryloxycarbonylderivat einer Aminosäure synthetisiert ist.

6. Verfahren nach Anspruch 5, dadurch gekennzeichnet, daß das Zwischenpeptid zu Beginn aus einem N^{ω}-Aryloxycarbonylderivat einer Diaminosäure synthetisiert ist.

7. Verfahren nach einem der Ansprüche 1 bis 4, dadurch gekennzeichnet, daß das Zwischenpeptid zu Beginn aus einer Peptidvorstufe durch Einführen einer Aryloxycarbonylgruppe an die in eine Carbamoylgruppe zu überführende Aminofunktion(en) innerhalb der Peptidkette synthetisiert wird.

8. Verbindung der allgemeinen Formel 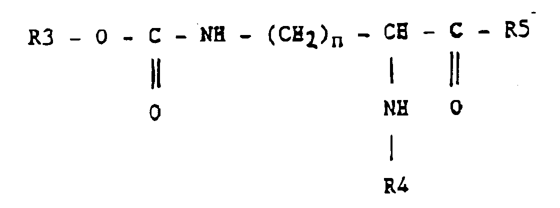 worin
- R3 eine Arylgruppe darstellt, die substituiert ist oder nicht mit einer oder mehreren Alkylgruppen, die 1 bis 4 Kohlenstoffatome umfassen,
- R4 ein Wasserstoffatom, eine Schutzgruppe für die Aminofunktion, eine Aminosäure oder ein Peptid darstellt, worin einige Funktionen wahlweise mit einer Schutzgruppe oder mit einer aktivierenden Gruppe substituiert sind,
- R5 eine Hydroxylgruppe, ein Halogenatom, eine Schutzgruppe für die Carboxylfunktion, eine aktivierende Gruppe, eine Aminogruppe, eine Aminosäure oder ein Peptid darstellt, worin einige Funktionen wahlweise mit einer Schutzgruppe oder mit einer aktivierenden Gruppe substituiert sind,
- n eine ganze Zahl von 1 bis 10 ist.

9. Verbindung nach Anspruch 8, dadurch gekennzeichnet, daß R3 eine Phenyl- oder p-Tolylgruppe ist und n eine ganze Zahl von 2 bis 4 ist.

10. Peptid der allgemeinen Formel
R6-Ser(R7)-Tyr(R8)-R9-R10
worin
- R6 ein Wasserstoffatom oder eine Schutzgruppe für die Aminofunktion ist,
- Ser(R7) ein Serinrest ist, wobei R7 ein Wasserstoffatom oder eine Schutzgruppe für die Hydroxylfunktion der Seitenkette des Serinrestes darstellt,
- Tyr(R8) ein Tyrosinrest ist, wobei R8 ein Wasserstoffatom oder eine Schutzgruppe für die Hydroxylfunktion der Seitenkette des Tyrosinrestes darstellt,
- R9 in einer ersten Variante ein 2,4-Diaminobuttersäure- oder Ornithinrest der Konfiguration D, L oder DL ist und in einer zweiten Variante ein 2-Amino-4-ureidobuttersäure-, Citrullin- oder Homocitrullinrest der Konfiguration D, L oder DL ist, worin die Wasserstoffatome der Carbamoylfunktion wahlweise substituiert sind durch eine Alkyl-, Cycloalkyl- oder Aralkylgruppe, die maximal 12 Kohlenstoffatome enthält, oder durch eine Cycloalkylengruppe, die 3 bis 6 Kohlenstoffatome enthält,
- R10 ein Hydroxylrest, eine Aminosäure oder ein Peptid ist, worin einige Funktionen wahlweise mit einer Schutzgruppe substituiert sind.
